# EUROPÄISCHE PATENTSCHRIFT

(11) **EP 3 574 842 B1**
(45) Veröffentlichungstag und Bekanntmachung des Hinweises auf die Patenterteilung: **21.12.2022**
(21) Anmeldenummer: 19172761.9
(22) Anmeldetag: 06.05.2019
(51) Int. Cl.: A61B 10/00, B01L 3/00, B67B 7/18, B65D 55/02, G01N 35/04, G01N 1/00, G09F 3/00

(54) **ÖFFNUNGSMASCHINENVORRICHTUNG UND PROBENBEHÄLTER ZUR VERWENDUNG MIT DER ÖFFNUNGSMASCHINENVORRICHTUNG**
OPENING MACHINE DEVICE AND SAMPLE CONTAINER FOR USE WITH THE OPENING MACHINE DEVICE
SYSTÈME D'OUVREUSE ET RÉCIPIENT À ÉCHANTILLONS DESTINÉ À ÊTRE UTILISÉ AVEC LE SYSTÈME D'OUVREUSE

(30) Priorität: 08.05.2018 DE 102018111064
(43) Veröffentlichungstag der Anmeldung: 04.12.2019
(73) Patentinhaber: Lockcon AG, 9050 Appenzell (CH)
(72) Erfinder: Lüdke, Flavio, 6345 Neuheim (CH)
(74) Vertreter: Daub, Thomas

(56) Entgegenhaltungen:
- EP-A1- 3 539 894
- WO-A1-2004/085280
- DE-A1-102016 120 726
- US-A- 4 630 743
- US-A- 4 674 340
- US-B1- 6 203 069

## Beschreibung

### Stand der Technik

Die Erfindung betrifft eine Öffnungsmaschinenvorrichtung nach dem Oberbegriff des Anspruchs 1 und ein Verfahren mit einer Öffnungsmaschinenvorrichtung nach dem Oberbegriff des Anspruchs 7.

Aus dem Stand der Technik sind bereits Öffnungsmaschinenvorrichtungen bekannt, welche dazu vorgesehen sind, eine automatische Öffnung von Probenbehältern bereitzustellen.

Die US 4 674 340 A offenbart eine Maschine zu einem Testen eines Drehmoments, welches zu einem Öffnen eines Behälters mit Schraubverschluss notwendig ist.

Die DE 10 2016 120726 A1 offenbart eine Öffnungsvorrichtung zur automatisierten Öffnung von Probenbehältern, wobei die Probenbehälter zur besseren Übersicht und Prozesskontrolle optische oder elektronische Identifizierungsmittel aufweisen.

Aus der US 4 630 743 A kennt man bereits einen Behälter mit einem Schraubverschluss, welcher manipulationssicher und kindersicher ist.

Die EP 3 539 894 A1 offenbart einen Behälter für die Verpackung fluider und/oder pastöser Substanzen, beispielsweise für Anwendungen im Haushalt, in Landwirtschaft, Industrie und Gewerbe.

Die WO 2004/085280 A1 offenbart einen Behälter zur Aufnahme von Flüssigkeiten wie beispielsweise Schmierölen oder Chemikalien.

Die US 6 203 069 B1 beschreibt ein Label mit einem unsichtbaren Barcode.

Die Aufgabe der Erfindung besteht insbesondere darin, eine gattungsgemäße Vorrichtung mit verbesserten Eigenschaften bezüglich einer hohen Sicherheit bereitzustellen. Die Aufgabe wird erfindungsgemäß durch die Merkmale der Ansprüche 1 und 7 gelöst, während vorteilhafte Ausgestaltungen und Weiterbildungen der Erfindung den Unteransprüchen entnommen werden können.

### Vorteile der Erfindung

Die Erfindung geht aus von einer Öffnungsmaschinenvorrichtung mit zumindest einer Öffnungseinheit und mit einer Recheneinheit zur automatischen Öffnung zumindest eines Probenbehälters.

Die erfindungsgemäße Öffnungsmaschinenvorrichtung umfasst zumindest eine Verifikationseinheit, welche dazu vorgesehen ist, zumindest eine Verifikationseigenschaft des Probenbehälters automatisch zu überprüfen, wobei die Verifikationseigenschaft eine Eigenschaft des Probenbehälters ist, welche zumindest eine Information bezüglich einer Unversehrtheit des Probenbehälters und/oder einer Unversehrtheit einer Probe aufweist und wobei die Verifikationseinheit zumindest eine Belastungssensoreinheit aufweist, welche dazu vorgesehen ist, zumindest eine mechanische Verifikationseigenschaft des Probenbehälters zu detektieren, wobei die Belastungssensoreinheit einen Drehmomentsensor aufweist und die mechanische Verifikationseigenschaft des Probenbehälters das zu einer Rotation der Verschlusseinheit relativ zur Aufnahmeeinheit benötigte Drehmoment definiert, wobei die Belastungssensoreinheit eine Übertragungseinheit aufweist, welche zu einer Übertragung des steigenden Drehmoments an die Recheneinheit dient, wobei die Recheneinheit das gemessene Drehmoment stetig mit einem vordefinierten Drehmoment vergleicht und, sobald beide Drehmomente identisch sind, ein Signal zur Freigabe der Öffnung des Probenbehälters an die Öffnungseinheit überträgt.

Hierdurch können insbesondere eine hohe Sicherheit und insbesondere auch ein erhöhter Bedienkomfort erreicht werden. Vorteilhaft können durch Personal ausgeführte Prüfvorgänge entfallen. Besonders vorteilhaft können eine hohe Präzision und eine hohe Geschwindigkeit des Prüfvorgangs erreicht werden.

Unter einer "Öffnungsmaschinenvorrichtung" soll insbesondere eine Vorrichtung verstanden werden, welche zumindest dazu vorgesehen ist, eine vorteilhaft automatische Öffnung zumindest eines Probenbehälters bereitzustellen. Insbesondere ist die Öffnungsmaschinenvorrichtung ein Teil einer Öffnungsmaschine. Insbesondere kann die Öffnungsmaschinenvorrichtung die gesamte Öffnungsmaschine umfassen. Vorteilhaft weist die Öffnungsmaschinenvorrichtung zumindest eine Öffnungseinheit auf, welche bevorzugt zumindest ein bewegliches Öffnungselement aufweist, welches dazu vorgesehen ist, zumindest bei einem Öffnungsvorgang zumindest eine Kraft und bevorzugt zumindest ein Drehmoment auf den Probenbehälter zu übertragen. Beispielsweise könnte die Öffnungseinheit zumindest einen Elektromotor aufweisen. Beispielsweise könnte das Öffnungselement zumindest ein rotierbares Griffelement, welches bevorzugt zumindest ein verformbares Material aufweist, aufweisen. Beispielsweise könnte das Öffnungselement zumindest eine rotierbar gelagerte Elastomerkappe aufweisen, welche dazu vorgesehen ist, bei einem Öffnungsvorgang an einer Verschlusseinheit, insbesondere einem Deckel, eines Probenbehälters anzugreifen.

Unter einem "Probenbehälter" soll insbesondere ein Behälter verstanden werden, welcher dazu vorgesehen ist, zumindest eine feste und/oder flüssige Probe aufzunehmen und zu lagern. Bevorzugt ist der Probenbehälter dazu vorgesehen, eine Probe zur Durchführung eines Dopingtests aufzunehmen. Denkbar könnte der Probenbehälter dazu vorgesehen sein, eine Probe zur Durchführung eines Drogentests und/oder DNS-Tests aufzunehmen. Vorteilhaft weist der Probenbehälter zumindest einen Aufnahmeraum zu einer Aufnahme der Probe auf. Besonders vorteilhaft weist der Probenbehälter zumindest eine Verschlusseinheit und zumindest eine Aufnahmeeinheit auf, welche in zumindest einem geschlossenen Zustand des Probenbehälters den Aufnahmeraum vollständig nach außen abgrenzen. Beispielsweise könnte die Verschlusseinheit zumindest eine Verschlusskappe und/oder zumindest einen Schraubdeckel aufweisen. Beispielsweise könnte die Aufnahmeeinheit zumindest einen Hohlkörper mit zumindest einer Öffnung aufweisen. Bevorzugt weisen die Aufnahmeeinheit und/oder die Verschlusseinheit eine Grundform auf, welche als ein zu einer Seite offener Hohlzylinder ausgebildet ist. Vorteilhaft weist der Probenbehälter zumindest eine Testeinheit auf, welche zumindest ein Testmittel, bevorzugt einen Adulterationsstreifen, zu einem Testen der Probe aufweist. Vorteilhaft ist der Probenbehälter unzerstörbar gegenüber Stößen von mindestens 20 N, vorteilhaft gegenüber Stößen von mindestens 40 N und besonders vorteilhaft gegenüber Stößen von mindestens 60 N. Beispielsweise könnte der Probenbehälter zumindest ein Copolyester, insbesondere Tritan, aufweisen. Vorteilhaft zeigt der Probenbehälter bei einer Einwirkung von hochkonzentrierten Säuren und/oder Laugen sichtbare Veränderungen. Besonders vorteilhaft zeigt der Probenbehälter bei einer Einwirkung von hochkonzentriertem Chlor und/oder Fluor sichtbare Veränderungen. Bevorzugt zeigt der Probenbehälter bei einer Einwirkung von Temperaturen von über 80°C, vorteilhaft von über 100°C und besonders vorteilhaft von über 120°C und/oder Temperaturen von unter -80°C, vorteilhaft von unter -100°C und besonders vorteilhaft von unter -120°C sichtbare Veränderungen.

Unter einer "Verifikationseinheit" soll insbesondere ein Teil der Öffnungsmaschinenvorrichtung verstanden werden, welcher dazu vorgesehen ist, zumindest eine Verifikationseigenschaft des Probenbehälters zu überprüfen. Beispielsweise kann die Verifikationseinheit hierzu zumindest eine Sensoreinheit aufweisen. Unter einer "Verifikationseigenschaft" soll insbesondere eine Eigenschaft des Probenbehälters verstanden werden, welche zumindest eine Information bezüglich einer Echtheit des Probenbehälters und/oder der Probe aufweist. Insbesondere führt die Verifikationseinheit zumindest einen Prüfvorgang am Probenbehälter durch. Insbesondere sind sämtliche Prüfvorgänge der Verifikationseinheit zeitlich vor einer Öffnung des Probenbehälters durch die Öffnungsmaschinenvorrichtung angeordnet. Vorteilhaft führt die Öffnungsmaschinenvorrichtung die Öffnung des Probenbehälters nur durch, wenn der Prüfvorgang zumindest ein positives Ergebnis liefert. Besonders vorteilhaft überprüft die Öffnungsmaschinenvorrichtung die Verifikationseigenschaft während eines Öffnungsvorgangs des Probenbehälters und führt den Öffnungsvorgang nur bei einem positiven Ergebnis vollständig durch. Darunter, dass "der Prüfvorgang ein positives Ergebnis liefert" soll insbesondere verstanden werden, dass die Verifikationseigenschaft identisch zu einer vordefinierten Verifikationseigenschaft ist. Bevorzugt ist die vordefinierte Verifikationseigenschaft durch zumindest einen Herstellungsprozess des Probenbehälters definiert. Denkbar ist, dass eine Prüfeinheit, welche unabhängig von der Öffnungsvorrichtung sein könnte, den Prüfvorgang unabhängig von dem Öffnungsvorgang durchführen könnte. Besonders vorteilhaft gibt die Öffnungsmaschinenvorrichtung das Ergebnis des Prüfvorgangs zumindest haptisch und/oder optisch und/oder akustisch wieder. Vorteilhaft erhält die Verifikationseinheit bei der Überprüfung der Verifikationseigenschaft die Information. Beispielsweise kann die Information zumindest eine ID des Probenbehälters und/oder eine ID der Probe und/oder eine Unversehrtheit des Probenbehälters und/oder eine Unversehrtheit der Probe beinhalten. Denkbar ist, dass zumindest eine weitere Verifikationseigenschaft einer weiteren Vorrichtung zumindest eine Information bezüglich einer Position des Probenbehälters, vorteilhaft zumindest einer GPS-Position des Probenbehälters, aufweist. Vorteilhaft weist die Öffnungsmaschinenvorrichtung zumindest eine Übertragungseinheit auf, welche dazu vorgesehen ist, die Information an zumindest eine Datenbank zu übertragen, insbesondere eine Datenbank im Internet. Besonders vorteilhaft ist die Information durch zumindest eine Blockchain gesichert. Beispielsweise kann die Verifikationseigenschaft einer Form, einer Beschädigung, einer Farbe, einem Druck, einer mechanischen Spannung, einer optischen und/oder strukturellen Markierung und/oder einem zu einer Öffnung benötigten Drehmoment zugehörig sein. Bevorzugt weisen die Verschlusseinheit und die Aufnahmeeinheit miteinander korrespondierende Verifikationseigenschaften auf. Bevorzugt ist die Aufnahmeeinheit, insbesondere vollständig, transparent. Vorteilhaft ist die Verschlusseinheit, insbesondere vollständig, opak.

Darunter, dass ein Vorgang "automatisch" durchgeführt wird, soll insbesondere verstanden werden, dass der Vorgang während einer Durchführung unabhängig von einem Bediener ist. Insbesondere kann der Bediener zumindest eine manuelle Starthandlung zu einer Aktivierung des automatischen Vorgangs durchführen, woraufhin der automatische Vorgang unabhängig von weiteren manuellen Bedienhandlungen des Bedieners durchgeführt wird.

Ferner wird vorgeschlagen, dass die Verifikationseinheit zumindest eine optische Sensoreinheit aufweist, welche dazu vorgesehen ist, zumindest eine optische Verifikationseigenschaft des Probenbehälters zu detektieren. Unter einer "optischen Verifikationseigenschaft" soll insbesondere eine Verifikationseigenschaft verstanden werden, welche durch zumindest ein sichtbares Bauteil und/oder zumindest einen sichtbaren Teilbereich des Probenbehälters definiert ist und vorteilhaft durch zumindest eine gängige optische Prüfeinheit detektiert werden kann. Vorteilhaft weist der Probenbehälter zu einer Definition einer vordefinierten optischen Verifikationseigenschaft des Probenbehälters zumindest eine aufgedruckte Nummer und/oder zumindest einen aufgedruckten QR-Code und/oder zumindest ein Label und/oder zumindest eine holographische ID auf. Besonders vorteilhaft prüft die optische Sensoreinheit zu einer Detektion der optischen Verifikationseinheit zumindest eine aufgedruckte Nummer und/oder zumindest einen aufgedruckten QR-Code und/oder zumindest ein Label und/oder zumindest eine holographische ID des Probenbehälters. Insbesondere weist die optische Sensoreinheit zumindest einen optischen Sensor auf. Vorteilhaft weist der optische Sensor zumindest eine Kamera auf. Besonders vorteilhaft weist die optische Sensoreinheit zumindest eine Antriebseinheit zu einer Bewegung des optischen Sensors auf. Hierdurch kann insbesondere eine Sicherheit weiter erhöht werden. Vorteilhaft kann eine Erkennung von sichtbaren Änderungen des Probenbehälters aufgrund eines Betrugsversuches, beispielsweise durch Einstiche, Schnitte, Zersetzungen, Verbiegungen, Änderungen des Füllstands und/oder Bohrungen gewährleistet werden.

Besonders vorteilhaft entfallen weitere durch Personal ausgeführte Prüfvorgänge, beispielsweise Beobachtungen des Probenbehälters.

. Unter einer "mechanischen Verifikationseigenschaft" soll insbesondere eine Verifikationseigenschaft verstanden werden, die durch zumindest eine mechanische Größe des Probenbehälters definiert ist und vorteilhaft durch zumindest eine gängige mechanische Prüfeinheit detektiert werden kann. Vorteilhaft weist der Probenbehälter zu einer Definition einer vordefinierten mechanischen Verifikationseigenschaft des Probenbehälters zumindest einen vordefinierten Druck und/oder zumindest eine strukturelle Markierung und/oder zumindest ein vordefiniertes, zu einer Öffnung benötigtes Drehmoment und/oder zumindest eine vordefinierte mechanische Spannung auf. Besonders vorteilhaft prüft die Belastungssensoreinheit zu einer Detektion der mechanischen Verifikationseigenschaft zumindest einen Druck und/oder zumindest eine strukturelle Markierung und/oder zumindest ein zu einer Öffnung benötigtes Drehmoment und/oder zumindest eine mechanische Spannung des Probenbehälters. Vorteilhaft weist die Belastungssensoreinheit zumindest einen Kraftsensor und/oder zumindest einen Drucksensor und/oder zumindest einen Spannungssensor und/oder zumindest einen Gewichtssensor zur Messung einer mechanischen Spannung auf. Hierdurch kann insbesondere eine Sicherheit noch weiter erhöht werden. Vorteilhaft kann eine Erkennung von nicht sichtbaren Änderungen des Probenbehälters aufgrund eines Betrugsversuches, beispielsweise durch Temperaturschwankungen, unerlaubtes Öffnen des Probenbehälters und/oder Einleiten von Flüssigkeiten und/oder Gasen in den Aufnahmeraum gewährleistet werden. Besonders vorteilhaft entfallen weitere durch Personal ausgeführte Prüfvorgänge, beispielsweise Gewichtsmessungen des Probenbehälters.

Darüber hinaus wird vorgeschlagen, dass die Belastungssensoreinheit zumindest teilweise einstückig mit der Öffnungseinheit ausgeführt ist. Darunter, dass zwei Einheiten "zumindest teilweise einstückig" miteinander ausgeführt sind, soll in diesem Zusammenhang insbesondere verstanden werden, dass die beiden Einheiten zumindest ein gemeinsames Bauteil aufweisen. Insbesondere ist zumindest ein Kraftsensor der Belastungssensoreinheit einstückig mit der Öffnungseinheit ausgeführt. Hierdurch kann insbesondere eine einfache und platzsparende Ausgestaltung der Öffnungsmaschinenvorrichtung erreicht werden. Vorteilhaft können zusätzliche Bauteile der Belastungssensoreinheit zu einem Überprüfen eines zu einer Öffnung benötigtem Drehmoment entfallen.

Des Weiteren wird vorgeschlagen, dass die Verifikationseinheit zumindest eine Ausleseeinheit aufweist, welche dazu vorgesehen ist, zumindest eine elektronische Verifikationseigenschaft des Probenbehälters zu detektieren. Unter einer "elektronischen Verifikationseigenschaft" soll insbesondere eine Verifikationseigenschaft verstanden werden, welche durch zumindest ein elektronisches Bauteil definiert ist und vorteilhaft durch zumindest eine gängige elektronische Prüfeinheit detektiert werden kann. Vorteilhaft weist der Probenbehälter zu einer Definition einer vordefinierten elektronischen Verifikationseigenschaft des Probenbehälters zumindest ein RFID-Tag und/oder zumindest eine elektronische Speichereinheit und/oder zumindest ein beschreibbares elektronisches Medium auf. Besonders vorteilhaft prüft die Ausleseeinheit zu einer Detektion der elektronischen Verifikationseigenschaft des Probenbehälters zumindest ein RFID-Tag und/oder zumindest eine elektronische Speichereinheit und/oder zumindest ein beschreibbares elektronisches Medium des Probenträgers. Vorteilhaft weist die Ausleseeinheit zumindest eine Sendeeinheit und/oder zumindest eine Empfängereinheit zu einer Übertragung von Radiowellen und/oder elektromagnetischen Feldern auf. Beispielsweise prüft die Ausleseeinheit zumindest eine Verifikationseigenschaft des Probenbehälters, welche zumindest ein RFID-Tag und/oder zumindest eine elektronische Speichereinheit und/oder zumindest ein beschreibbares elektronisches Medium aufweist. Hierdurch kann insbesondere eine Sicherheit weiter erhöht und eine platzsparende Ausgestaltung der Öffnungsmaschinenvorrichtung erreicht werden. Vorteilhaft kann eine einzelne Verifikationseigenschaft eine Vielzahl von Informationen aufweisen. Besonders vorteilhaft entfallen weitere durch Personal durchgeführte Prüfvorgänge, beispielweise ein manuelles Bedienen eines Lesegeräts.

Zudem wird eine Öffnungsmaschine mit einer erfindungsgemäßen Öffnungsmaschinenvorrichtung vorgeschlagen, wodurch eine Sicherheit vorteilhaft erhöht werden kann.

Ferner wird ein Probenbehälter vorgeschlagen, welcher zur Verwendung mit einer erfindungsgemäßen Öffnungsmaschinenvorrichtung vorgesehen ist und welcher zumindest eine Aufnahmeeinheit und wenigstens eine mit der Aufnahmeeinheit in zumindest einem verschlossenen Zustand kooperierende Verschlusseinheit aufweist. Hierdurch können insbesondere eine Sicherheit und vorteilhaft eine erhöhter Bedienkomfort erreicht werden.

Vorteilhaft weist der Probenbehälter zumindest eine Sollbrucheinheit auf, welche dazu vorgesehen ist, nach einer ersten Rotation der Verschlusseinheit relativ zur Aufnahmeeinheit um eine Drehachse einen aktivierten Zustand und nach zumindest einem Öffnungsschritt einen zerstörten Zustand zu erreichen, und welche zumindest eine zu einem Großteil parallel zur Drehachse verlaufende Sollbruchkante aufweist. Unter einem "aktivierten Zustand der Sollbrucheinheit" soll insbesondere ein Zustand verstanden werden, in dem die Sollbrucheinheit bei einem Einwirken einer vordefinierten Kraft, insbesondere einem vordefinierten Drehmoment, in den zerstörten Zustand überführt wird. Vorteilhaft bewirkt die Kraft eine Bewegung der Verschlusseinheit relativ zu der Aufnahmeeinheit. Besonders vorteilhaft dient die Kraft der Öffnung des Probenbehälters. Unter einem "Öffnungsschritt" soll insbesondere ein Prozess verstanden werden, welcher eine Überführung der Sollbrucheinheit von dem aktivierten Zustand in den zerstörten Zustand bereitstellt. Insbesondere kann der Öffnungsschritt eine zweite Rotation der Verschlusseinheit relativ zur Aufnahmeeinheit um die Drehachse umfassen. Vorteilhaft weist der Öffnungsschritt zumindest einen Druckprozess und/oder zumindest einen Schneidprozess auf. Besonders vorteilhaft weist die Öffnungsmaschinenvorrichtung zumindest ein scharfes Öffnungselement, beispielsweise ein Messer, ein Scherelement o. dgl., zur Öffnung des Probenbehälters auf. Bevorzugt weist die Sollbrucheinheit zumindest eine Rasteinheit mit zumindest einem ersten und einem korrespondierenden zweiten Rastelement auf, welche im aktivierten Zustand der Sollbrucheinheit zumindest eine gemeinsame Rastverbindung bilden. Besonders bevorzugt ist das erste Rastelement an der Verschlusseinheit und das zweite Rastelement an der Aufnahmeeinheit angeordnet. Vorteilhaft ist ein Bilden der Rastverbindung haptisch und/oder akustisch und/oder optisch für den Bediener erkennbar. Unter einem "zerstörten Zustand der Sollbrucheinheit" soll insbesondere ein Zustand verstanden werden, welcher sich durch eine sichtbare mechanische Veränderung der Sollbrucheinheit von dem aktivierten Zustand unterscheidet. Beispielsweise kann die Veränderung eine zumindest teilweise Verformung der Sollbrucheinheit und/oder eine zumindest teilweise Trennung der Sollbrucheinheit von einem restlichen Probenbehälter umfassen. Insbesondere kann die Sollbrucheinheit im zerstörten Zustand vollständig von dem restlichen Probenbehälter getrennt sein. Insbesondere ist der zerstörte Zustand permanent. Bevorzugt dient die erste Rotation einem Verschließen des Probenbehälters. Besonders bevorzugt umfasst der Öffnungsschritt ein Öffnen des Probenbehälters. Insbesondere verläuft die Drehachse parallel zu einer Haupterstreckungsrichtung des Probenbehälters. Unter einer "Haupterstreckungsrichtung" eines Objekts soll dabei insbesondere eine Richtung verstanden werden, welche parallel zu einer längsten Kante eines kleinsten geometrischen Quaders verläuft, welcher das Objekt gerade noch vollständig umschließt. Insbesondere können die erste Rotation und die zweite Rotation über einen beliebigen Winkelbereich erfolgen. Vorteilhaft erfolgen die erste Rotation und die zweite Rotation über denselben Winkelbereich. Unter einer "Sollbruchkante" soll insbesondere ein Teilbereich der Sollbrucheinheit verstanden werden, welcher insbesondere die Sollbrucheinheit mit dem restlichen Probenbehälter verbindet und dazu vorgesehen ist, im zerstörten Zustand zumindest teilweise und optisch auffällig zertrennt zu sein. Insbesondere ist die Sollbrucheinheit von gängigen, als ineinandergreifende Nockenringe ausgebildeten Sollbrucheinheiten verschieden ausgebildet. Hierdurch können insbesondere eine Sicherheit und ein Bedienkomfort weiter verbessert werden. Vorteilhaft können Änderungen des Probenbehälters aufgrund von zumindest einem unerlaubten Öffnen des Probenbehälters auf einfache Weise sichtbar gemacht werden. Besonders vorteilhaft kann eine intuitiv bedienbare Ausgestaltung der Sollbrucheinheit erreicht werden, insbesondere ist ein Überführen der Sollbrucheinheit in den aktivierten Zustand für den Bediener intuitiv ausführbar und deutlich erkennbar.

Besonders vorteilhaft weist der Probenbehälter zumindest eine lediglich unter Verwendung zumindest einer Sehhilfe erkennbare Markierung auf, welche die Verifikationseigenschaft aufweist. Unter einer "Sehhilfe" soll insbesondere eine externe Einheit verstanden werden, welche dazu dient, mit bloßem Auge bei natürlichem Licht unsichtbare Markierungen sichtbar zu machen. Beispielsweise könnte die Sehhilfe eine Infrarot-Lichtquelle und/oder eine UV-Lichtquelle und/oder einen Laser und/oder eine optische Vergrößerungseinheit aufweisen. Vorteilhaft weist die Öffnungsmaschinenvorrichtung zumindest eine Sehhilfe auf. Denkbar ist, dass eine Vielzahl von Markierungen mit jeweils unterschiedlicher Ausgestaltung existiert, wobei vordefinierte Gruppen von Probenbehältern jeweils identische Markierungen aufweisen. Beispielsweise kann die Markierung zumindest ein UV-Tag aufweisen. Hierdurch kann insbesondere eine Sicherheit noch weiter erhöht werden. Vorteilhaft kann die Markierung geheim gehalten und damit vor Betrugsversuchen geschützt werden.

Ferner wird vorgeschlagen, dass der Probenbehälter zumindest eine Außenwandung aufweist, an welcher die Markierung angeordnet ist. Unter einer "Außenwandung" soll insbesondere ein Teilbereich des Probenbehälters verstanden werden, welcher den Aufnahmeraum nach außen begrenzt und von außen sichtbar ist. Insbesondere umfasst die Außenwandung eine Seitenfläche und einen Boden der Aufnahmeeinheit sowie eine Seitenfläche und eine Oberseite der Verschlusseinheit. Unter "angeordnet" kann in diesem Fall insbesondere verstanden werden, dass die Markierung auf die Außenwandung aufgetragen und/oder in die Außenwandung eingebettet ist. Beispielsweise könnte die Markierung zumindest ein während der Herstellung des Probenbehälters in ein Material der Aufnahmeeinheit gemischtes weiteres Material aufweisen. Hierdurch kann insbesondere eine Prüfung der Markierung vereinfacht werden.

Vorteilhaft können Änderungen des Probenbehälters aufgrund von zumindest einem Eindringen eines Fremdkörpers, beispielsweise einer Nadel und/oder einem Messer, von außen in den Aufnahmeraum auf einfache Weise sichtbar gemacht werden, wenn die Außenwandung in sämtlichen Teilbereichen zumindest zwei Wandungen umfasst. Insbesondere muss der Fremdkörper zum Eindringen von außen in den Aufnahmeraum zumindest eine äußere Wandung und zumindest eine innere Wandung durchdringen. Bevorzugt weist der Probenbehälter zwischen der Seitenfläche der Aufnahmeeinheit und einem Halsbereich der Aufnahmeeinheit zumindest eine Nut auf, insbesondere um einen spaltlosen Übergang der Au-ßenwandung von der Aufnahmeeinheit zu der Verschlusseinheit zu erreichen. Insbesondere ist zwischen den Wandungen zumindest ein leerer, beispielsweise mit Luft gefüllter, Leerraum angeordnet. Denkbar wäre, dass der Leerraum mit einem transparenten Material gefüllt ist, welches dazu vorgesehen ist, das Eindringen des Fremdkörpers noch leichter erkennbar zu machen, beispielsweise könnte der Leerraum mit einem Gas gefüllt sein, welches nach dem Eindringen des Fremdkörpers entweicht und/oder seine Konzentration ändert. Vorteilhaft sind auf zumindest einer Ebene, welche senkrecht zu einer Höhenrichtung der Aufnahmeeinheit verläuft und den Halsbereich der Aufnahmeeinheit schneidet, die beiden Wandungen durch die Verschlusseinheit und den Halsbereich der Aufnahmeeinheit gebildet. Besonders vorteilhaft weist zumindest eine der Wandungen eine, bevorzugt transparente, Beschichtung auf, welche insbesondere nach dem Eindringen des Fremdkörpers durch die Wandung zumindest eine Beschädigung aufweist, wobei die Beschädigung zumindest durch die Öffnungsmaschine und bevorzugt durch den Bediener erkennbar ist. Bevorzugt weist die Beschichtung zumindest ein Glas auf, besonders bevorzugt ist die Beschichtung vollständig aus Glas gebildet. Hierdurch kann insbesondere eine Sicherheit weiter erhöht werden. Vorteilhaft kann nach dem Eindringen des Fremdkörpers ein Wiederherstellen der Außenwand in einen Zustand vor dem Eindringen des Fremdkörpers zusätzlich erschwert werden.

Weiterhin wird vorgeschlagen, dass die Aufnahmeeinheit zumindest ein Formschlusselement aufweist, welches dazu vorgesehen ist, mit einem korrespondierenden Formschlusselement eines weiteren Probenbehälters zusammenzuwirken. Insbesondere können das Formschlusselement und das korrespondierende Formschlusselement einen gemeinsamen Formschluss bilden. Insbesondere dient der gemeinsame Formschluss einer vordefinierten Anordnung des Probenbehälters an einem benachbarten weiteren Probenbehälter. Beispielsweise könnte der gemeinsame Formschluss ein Stapeln und/oder ein Aneinanderreihen von mehreren Probenträgern vereinfachen. Vorteilhaft ist das Formschlusselement an dem Boden der Aufnahmeeinheit angeordnet. Denkbar ist, dass das Formschlusselement an der Seitenfläche der Aufnahmeeinheit angeordnet ist. Bevorzugt umfasst das Formschlusselement zumindest eine Erhebung einer Außenfläche der Aufnahmeeinheit. Besonders bevorzugt umfasst das korrespondierende Formschlusselement zumindest eine weitere Erhebung einer weiteren Außenfläche einer weiteren Aufnahmeeinheit des weiteren Probenbehälters, welche formschlüssig in die Erhebung eingreifen kann. Hierdurch kann insbesondere ein Bedienkomfort weiter gesteigert werden. Vorteilhaft können eine Vielzahl von Probenbehältern platzsparend gelagert werden.

Darüber hinaus wird vorgeschlagen, dass die Verschlusseinheit zumindest ein weiteres Formschlusselement aufweist, welches identisch zu dem korrespondierenden Formschlusselement ausgestaltet ist. Vorteilhaft ist das Formschlusselement an der Oberseite der Verschlusseinheit angeordnet. Denkbar ist, dass das Formschlusselement an der Seitenfläche der Verschlusseinheit angeordnet ist. Hierdurch kann insbesondere ein Bedienkomfort weiter gesteigert werden. Vorteilhaft können eine Vielzahl von Probenbehältern unabhängig von einer Reihenfolge der Probenbehälter in einer Reihe und/oder auf einem Stapel platzsparend gelagert werden.

Die Erfindung geht ferner aus von einem Verfahren mit einer Öffnungsmaschinenvorrichtung, bei welchem zumindest ein Probenbehälter automatisch geöffnet wird. Es wird vorgeschlagen, dass zumindest eine Verifikationseigenschaft des Probenbehälters automatisch überprüft wird. Hierdurch können insbesondere eine Sicherheit und ein Bedienkomfort erhöht werden.

Die erfindungsgemäße Öffnungsmaschinenvorrichtung und das erfindungsgemäße Verfahren sollen hierbei nicht auf die oben beschriebene Anwendung und Ausführungsform beschränkt sein. Insbesondere können die Öffnungsmaschinenvorrichtung und das Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: Eine schematische Darstellung einer Öffnungsmaschine mit einer Öffnungsmaschinenvorrichtung und einen Probenbehälter nach einem ersten Verschließen,
- Fig. 2: Eine Querschnittsdarstellung des geschlossenen Probenbehälters entlang einer Schnittlinie A in Fig. 1,
- Fig. 3: eine Querschnittsdarstellung des geschlossenen Probenbehälters entlang einer Schnittlinie B in Fig. 1,
- Fig. 4: den Probenbehälter vor dem ersten Verschließen in einer Draufsicht,
- Fig. 5: den Probenbehälter vor dem ersten Verschließen in einer Schrägansicht,
- Fig. 6: den Probenbehälter nach einem Öffnen,
- Fig. 7: ein Verlaufsdiagramm zur Darstellung eines Verfahrens mit der Öffnungsmaschinenvorrichtung,
- Fig. 8: eine Querschnittsdarstellung eines weiteren, geschlossenen Probenbehälters entlang einer Schnittlinie C in Fig. 10,
- Fig. 9: eine weitere Querschnittsdarstellung des weiteren, geschlossenen Probenbehälters entlang einer Schnittlinie D in Fig. 8,
- Fig. 10: den weiteren Probenbehälter vor einem ersten Verschließen in einer Schrägansicht,
- Fig. 11: den weiteren geschlossenen Probenbehälter in einer ersten Schrägansicht und
- Fig. 12: den weiteren geschlossenen Probenbehälter in einer zweiten Schrägansicht.

### Beschreibung der Ausführungsbeispiele

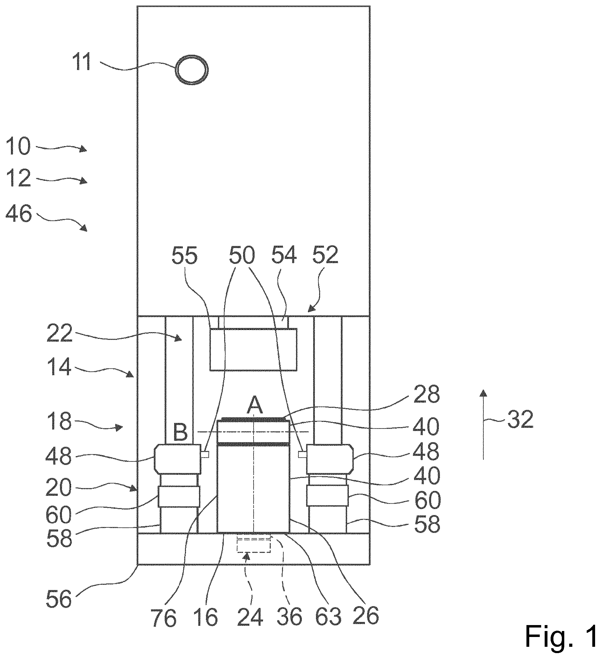

Fig. 1 zeigt ein System 46. Das System 46 weist eine Öffnungsmaschine 10 auf. Das System 46 weist einen Probenbehälter 16 auf. Der Probenbehälter 16 weist eine Aufnahmeeinheit 26 auf. Der Probenbehälter 16 weist eine Verschlusseinheit 28 auf. Der Probenbehälter 16 befindet sich in einem geschlossenen Zustand. Die Öffnungsmaschine 10 weist eine Öffnungsmaschinenvorrichtung 12 auf. Die Öffnungsmaschinenvorrichtung 12 weist einen Schalter 11 auf. Die Öffnungsmaschinenvorrichtung 12 weist eine Plattform 56 auf. Die Öffnungsmaschinenvorrichtung 12 weist zwei Haltestangen 58 auf. Die Haltestangen 58 sind in der Plattform 56 verankert. Alternativ könnte die Öffnungsmaschinenvorrichtung 12 eine beliebige Anzahl weiterer Haltestangen aufweisen. Die Öffnungsmaschinenvorrichtung 12 weist eine Recheneinheit (nicht dargestellt) auf. Die Öffnungsmaschinenvorrichtung 12 weist eine Öffnungseinheit 14 auf. Die Öffnungseinheit 14 ist zu einer automatischen Öffnung des Probenbehälters 16 vorgesehen. Die Öffnungsmaschinenvorrichtung 12 weist eine Verifikationseinheit 18 auf. Die Verifikationseinheit 18 ist dazu vorgesehen, mehrere Verifikationseigenschaften des Probenbehälters 16 automatisch zu überprüfen.

Die Öffnungseinheit 14 weist zwei Halteelemente 48 auf. Die Halteelemente 48 weisen jeweils ein Ausfahrelement 50 auf. Die Ausfahrelemente 50 sind dazu vorgesehen, bei einem Öffnungsvorgang durch eine automatische translatorische Bewegung der Ausfahrelemente 50 den Probenbehälter 16 zwischen den Ausfahrelementen 50 zu fixieren. Alternativ könnte die Öffnungseinheit 14 eine Vielzahl weiterer Halteelemente aufweisen. Alternativ könnte der Probenbehälter 16 durch eine manuelle translatorische Bewegung der Ausfahrelemente 50 zwischen den Ausfahrelementen 50 fixiert sein. Die Öffnungsmaschinenvorrichtung 12 weist ein Öffnungselement 52 auf. Das Öffnungselement 52 weist ein weiteres Ausfahrelement 54 auf. Das Öffnungselement 52 weist eine Kappe 55 auf. Die Öffnungseinheit 14 weist einen Elektromotor (nicht dargestellt) auf. Der Elektromotor ist zu einer Bereitstellung einer translatorischen Bewegung der Ausfahrelemente 50, 54 vorgesehen. Der Elektromotor ist zu einer Bereitstellung einer Rotationsbewegung der Kappe 55 vorgesehen.

Die Verifikationseinheit 18 weist eine optische Sensoreinheit 20 auf. Die optische Sensoreinheit 20 ist dazu vorgesehen, eine optische Verifikationseigenschaft des Probenbehälters 16 zu detektieren. Die optische Verifikationseigenschaft weist eine Information über eine Beschädigung des Probenbehälters 16 auf. Die optische Sensoreinheit 20 weist zwei Kameras 60 auf. Alternativ könnte die optische Sensoreinheit 20 eine Vielzahl weiterer Kameras aufweisen. Die Kameras 60 sind an den Haltestangen 58 gehaltert. Die Kameras 60 sind dazu vorgesehen, bei einem Prüfvorgang der optischen Verifikationseigenschaft jeweils ein Bild des Probenbehälters 16 aufzunehmen. Die optische Sensoreinheit 20 weist eine Übertragungseinheit (nicht dargestellt) auf. Die Übertragungseinheit dient einer Übertragung der Bilder an die Recheneinheit. Die Recheneinheit erkennt durch die Bilder die optische Verifikationseigenschaft des Probenbehälters 16. Bei einem Prüfvorgang vergleicht die Recheneinheit die optische Verifikationseigenschaft mit einer vordefinierten optischen Verifikationseigenschaft. Sind beide optischen Verifikationseigenschaften identisch, so überträgt die Recheneinheit ein Signal zu einer Freigabe der Öffnung des Probenbehälters 16 an die Öffnungseinheit 14.

Die Verifikationseinheit 18 weist eine Belastungssensoreinheit 22 auf. Die Belastungssensoreinheit 22 ist dazu vorgesehen, eine mechanische Verifikationseigenschaft des Probenbehälters 16 zu detektieren. Die mechanische Verifikationseigenschaft weist eine Information über ein unerlaubtes Öffnen des Probenbehälters 16 auf. Die Belastungssensoreinheit 22 ist teilweise einstückig mit der Öffnungseinheit 14 ausgeführt. Die Belastungssensoreinheit 22 weist einen Drehmomentsensor (nicht dargestellt) auf. Der Drehmomentsensor ist einstückig mit der Kappe 55 ausgeführt. Der Drehmomentsensor misst ein Drehmoment der Kappe 55. Der Elektromotor stellt während eines Prüfvorgangs ein steigendes Drehmoment der Kappe 55 bereit. Das zu einer Rotation der Verschlusseinheit 28 relativ zur Aufnahmeeinheit 26 benötigte Drehmoment definiert die mechanische Verifikationseigenschaft des Probenbehälters 16. Die Belastungssensoreinheit 22 weist eine weitere Übertragungseinheit (nicht dargestellt) auf. Die weitere Übertragungseinheit dient einer Übertragung des steigenden Drehmoments an die Recheneinheit. Die Recheneinheit vergleicht das gemessene Drehmoment stetig mit einem vordefinierten Drehmoment. Sobald beide Drehmomente identisch sind, überträgt die Recheneinheit ein Signal zu einer Freigabe der Öffnung des Probenbehälters 16 an die Öffnungseinheit 14.

Die Verifikationseinheit 18 weist zumindest eine Ausleseeinheit 24 auf. Die Ausleseeinheit 24 ist dazu vorgesehen, eine elektronische Verifikationseigenschaft des Probenbehälters 16 zu detektieren. Die elektronische Verifikationseigenschaft weist eine Information über eine Identität des Probenbehälters 16 auf. Der Probenbehälter 16 weist ein RFID-Tag 61 auf. Das RFID-Tag 61 ist auf einem Boden 63 des Probenbehälters 16 aufgeklebt. Das RFID-Tag 61 definiert die elektronische Verifikationseigenschaft des Probenbehälters 16. Die Ausleseeinheit 24 umfasst ein gängiges, zu einem Auslesen von RFID-Tags vorgesehenes Ausleseelement (nicht dargestellt). Das Ausleseelement kann beispielsweise eine Antenne aufweisen. Die Ausleseeinheit 24 erhält bei einem Prüfvorgang der elektronischen Verifikationseigenschaft die Information durch das RFID-Tag 61. Die Ausleseeinheit 24 weist eine weitere Übertragungseinheit (nicht dargestellt) auf. Die weitere Übertragungseinheit dient einer Übertragung der Information des RFID-Tags 61 an die Recheneinheit. Die Recheneinheit vergleicht die Information mit einer vordefinierten Information. Sind beide Informationen identisch, so überträgt die Recheneinheit ein Signal zu einer Freigabe der Öffnung des Probenbehälters 16 an die Öffnungseinheit 14.

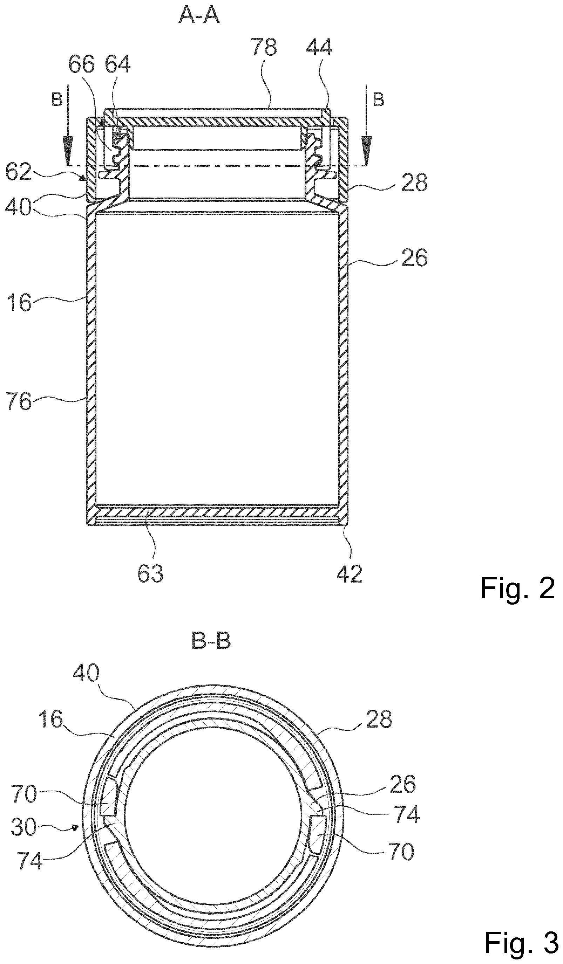

Fig. 2 und 3 zeigen Querschnittsdarstellungen des geschlossenen Probenbehälters 16. Die Verschlusseinheit 28 kooperiert mit der Aufnahmeeinheit 26. Die Verschlusseinheit 28 ist als ein zylindrischer Schraubdeckel ausgebildet. Die Aufnahmeeinheit 26 ist als ein zylindrischer Behälter ausgebildet. Die Aufnahmeeinheit 26 weist einen Halsbereich 62 auf. Die Aufnahmeeinheit 26 weist in dem Halsbereich 62 einen geringeren Durchmesser auf als außerhalb des Halsbereichs 62. Der Halsbereich 62 weist ein Gewinde 64 auf. Die Verschlusseinheit 28 weist ein mit dem Gewinde 64 kooperierendes weiteres Gewinde 66 auf.

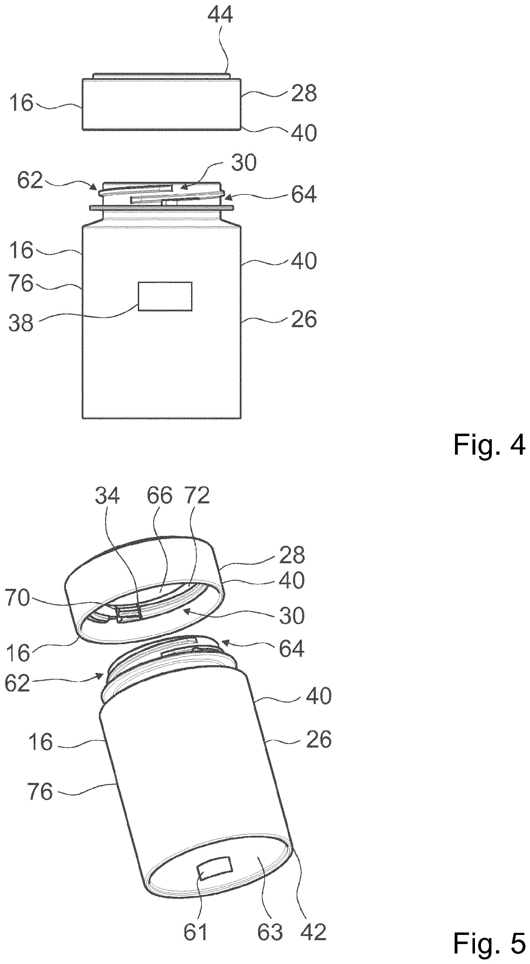

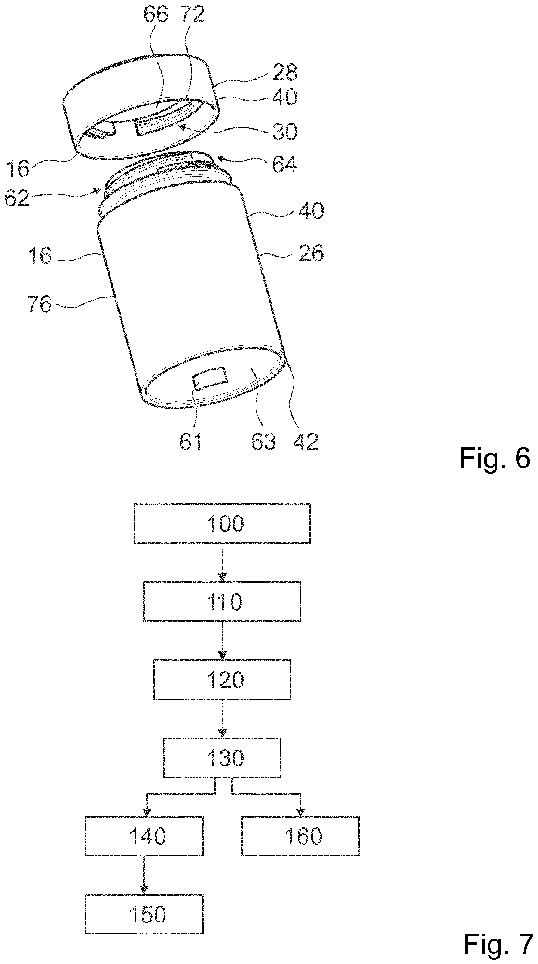

Der Probenbehälter 16 weist eine Sollbrucheinheit 30 auf. Die Sollbrucheinheit 30 ist in Fig. 4 und 5 deutlich dargestellt. Die Sollbrucheinheit 30 ist dazu vorgesehen, nach einer ersten Rotation der Verschlusseinheit 28 relativ zur Aufnahmeeinheit 26 um eine Drehachse 32 einen aktivierten Zustand zu erreichen. Die Sollbrucheinheit 30 ist dazu vorgesehen, nach einem Öffnungsschritt einen zerstörten Zustand zu erreichen. Der Öffnungsschritt umfasst eine zweite Rotation der Verschlusseinheit 28 relativ zur Aufnahmeeinheit 26 um die Drehachse 32. Alternativ könnte der Öffnungsschritt einen Druckprozess und/oder einen Schneidprozess umfassen. Der zerstörte Zustand der Sollbrucheinheit 30 ist in Fig. 6 sichtbar. Die Sollbrucheinheit 30 weist eine zu einem Großteil parallel zur Drehachse 32 verlaufende Sollbruchkante 34 auf. Im zerstörten Zustand ist die Sollbruchkante 34 vollständig zertrennt. Die Sollbrucheinheit 30 weist zwei Sollbruchelemente 70 auf. Die Sollbruchelemente 70 sind zueinander identisch, weshalb im Folgenden nur eines der Sollbruchelemente 70 beschrieben wird. Das Sollbruchelement 70 ist an einer Innenwand 72 der Verschlusseinheit 28 angeordnet. Im zerstörten Zustand ist das Sollbruchelement 70 vollständig von einem restlichen Probenbehälter 16 getrennt. Alternativ könnte das Sollbruchelement 70 im zerstörten Zustand teilweise von dem restlichen Probenbehälter 16 getrennt sein. Die Sollbrucheinheit 30 weist zwei Rastelemente 74 auf. Die Rastelemente 74 sind zueinander identisch, weshalb im Folgenden nur eines der Rastelemente 74 beschrieben wird. Das Rastelement 74 ist am Halsbereich 62 des Probenbehälters 16 angeordnet. Das Rastelement 74 bildet im aktivierten Zustand eine gemeinsame Rastverbindung mit dem Sollbruchelement 70 aus. Die gemeinsame Rastverbindung verbindet das Rastelement 74 zerstörungsfrei unlösbar mit dem Sollbruchelement 70.

Die Öffnungsmaschinenvorrichtung 12 weist eine Sehhilfe 36 auf. Der Probenbehälter 16 weist eine lediglich unter Verwendung der Sehhilfe 36 erkennbare Markierung 38 auf. Die Markierung 38 definiert eine weitere optische Verifikationseigenschaft. Der Probenbehälter 16 weist eine Außenwandung 40 auf. Die Markierung 38 ist an der Außenwandung 40 angeordnet. Die Markierung 38 ist als ein UV-Tag ausgebildet. Die Markierung 38 ist an einer Seitenfläche 76 der Aufnahmeeinheit 26 angeordnet. Aus Gründen der Übersichtlichkeit ist die Markierung 38 lediglich in Fig. 4 dargestellt. Die Sehhilfe 36 ist als eine UV-Lichtquelle ausgebildet. Die Sehhilfe 36 ist in einer Ausnehmung einer Oberseite (nicht dargestellt) der Plattform 56 angeordnet. Die Sehhilfe 36 ist dazu vorgesehen, die Markierung 38 während des Prüfvorgangs der optischen Verifikationseigenschaft des Probenbehälters 16 sichtbar zu machen. Bei einer Einwirkung von von der Sehhilfe 36 bereitgestelltem UV-Licht verfärbt sich die Markierung 38. Die optische Sensoreinheit 20 prüft die weitere optische Verifikationseigenschaft analog zum Prüfvorgang der optischen Verifikationseigenschaft.

Die Aufnahmeeinheit 26 weist ein Formschlusselement 42 auf. Das Formschlusselement 42 ist dazu vorgesehen, mit einem korrespondierenden Formschlusselement eines weiteren Probenbehälters zusammenzuwirken. Die Verschlusseinheit 28 weist ein weiteres Formschlusselement 44 auf. Das weitere Formschlusselement 44 ist identisch zu dem korrespondierenden Formschlusselement ausgestaltet. Das Formschlusselement 42 ist als ein kreisförmiger Steg ausgebildet. Das Formschlusselement 42 ist an dem Boden 63 der Aufnahmeeinheit 26 angeordnet. Das weitere Formschlusselement 44 ist an einer Oberseite 78 der Verschlusseinheit 28 angeordnet. Das weitere Formschlusselement 44 ist als eine kreisförmige Erhebung ausgebildet. Alternativ könnten die Formschlusselemente 42, 44 auch als Rastelemente ausgebildet sein. Alternativ könnten die Formschlusselemente 42, 44 an beliebigen anderen Stellen des Probenbehälters 16 angeordnet sein. Das Formschlusselement 42 ist dazu geeignet, das weitere Formschlusselement 44 formschlüssig aufzunehmen.

Fig. 7 zeigt ein schematisches Verlaufsdiagramm eines Verfahrens mit der Öffnungsmaschinenvorrichtung 12. In einem ersten Verfahrensschritt 100 wird der geschlossene Probenbehälter 16 zwischen den Haltestangen 58 auf der Plattform 56 positioniert. In einem zweiten Verfahrensschritt 110 wird der Schalter 11 durch den Bediener betätigt. Der zweite Verfahrensschritt 110 folgt hierbei auf den ersten Verfahrensschritt 100. In einem dritten Verfahrensschritt 120 stellt der Elektromotor eine translatorische Bewegung der Ausfahrelemente 50, 54 bereit. Die Ausfahrelemente 50 fixieren den Probenbehälter 16. Das Ausfahrelement 54 stellt einen Formschluss der Verschlusseinheit 28 mit der Kappe 55 bereit. Der dritte Verfahrensschritt 120 folgt hierbei auf den zweiten Verfahrensschritt 110. In einem vierten Verfahrensschritt 130 detektieren die optische Sensoreinheit 20, die Belastungssensoreinheit 22 und die Ausleseeinheit 24 Verifikationseigenschaften des Probenbehälters 16. Während eines Prüfvorgangs ist die Sehhilfe 36 aktiviert. Der vierte Verfahrensschritt 130 folgt hierbei auf den dritten Verfahrensschritt 120. Wenn die geprüften Verifikationseigenschaften zu den vordefinierten Verifikationseigenschaften identisch sind, erfolgt ein fünfter Verfahrensschritt 140. Im fünften Verfahrensschritt 140 stellt der Elektromotor eine Rotationsbewegung der Kappe 55 bereit. Die Rotationsbewegung trennt die Gewinde 64, 66 voneinander. Der fünfte Verfahrensschritt 140 folgt hierbei auf den vierten Verfahrensschritt 130. In einem sechsten Verfahrensschritt 150 stellt der Elektromotor weitere translatorische Bewegungen der Ausfahrelemente 50, 54 bereit. Die translatorische Bewegung des Ausfahrelements 54 hebt die Verschlusseinheit 28 von der Aufnahmeeinheit 26. Die translatorische Bewegung der Ausfahrelemente 50 löst die Fixierung des Probenbehälters 16. Wenn zumindest eine der geprüften Verifikationseigenschaften zu der jeweiligen vordefinierten Verifikationseigenschaft verschieden ist, erfolgt auf den vierten Verfahrensschritt 130 ein siebter Verfahrensschritt 160. Im siebten Verfahrensschritt 160 gibt eine Ausgabeeinheit (nicht dargestellt) der Öffnungsmaschinenvorrichtung 12 ein optisches Signal aus. Das optische Signal ist als ein Warnsignal ausgebildet.

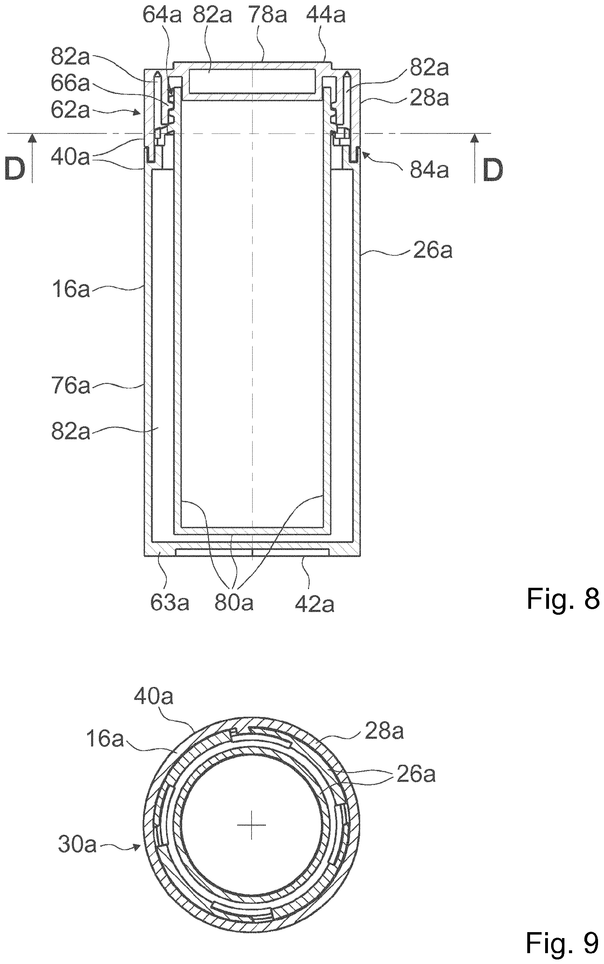

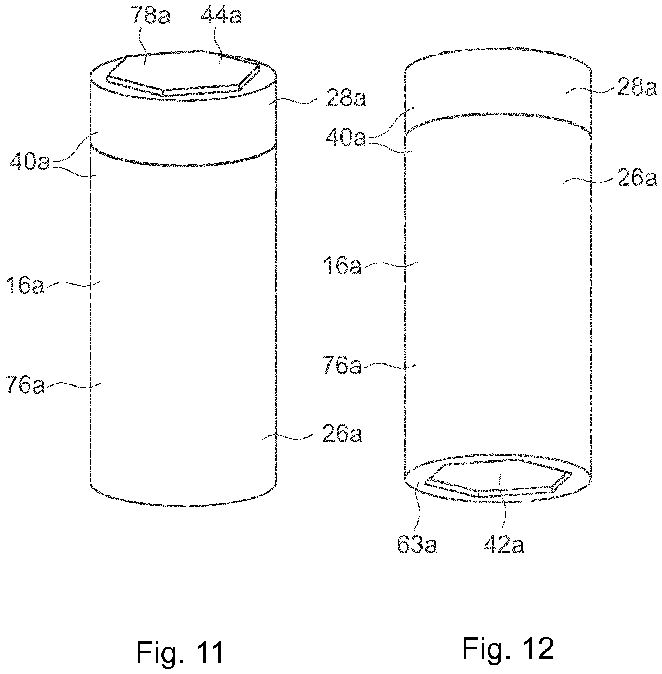

In den Fig. 8 bis 12 ist ein weiteres Ausführungsbeispiel der Erfindung gezeigt. Die nachfolgende Beschreibung und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Fig. 1 bis 7, verwiesen werden kann. Zur Unterscheidung der Ausführungsbeispiele ist der Buchstabe a den Bezugszeichen des Ausführungsbeispiels in Fig. 8 bis 12 nachgestellt.

Fig. 8 und 9 zeigen Querschnittsdarstellungen eines weiteren Probenbehälters 16a. Die Außenwandung 40a des weiteren Probenbehälters 40a umfasst in sämtlichen Teilbereichen zumindest zwei Wandungen. Auf einer Ebene des Halsbereichs 62a sind beide Wandungen durch die Verschlusseinheit 28a und den Halsbereich 62a gebildet. Die Seitenfläche 76a, der Boden 63a und die Oberseite 78a weisen jeweils eine Innenwandung 80a auf. Die Innenwandungen 80a und der Halsbereich 62a weisen jeweils eine Beschichtung (nicht dargestellt) auf. Die Beschichtungen sind dazu vorgesehen, nach einem Eindringen eines Fremdkörpers durch eine der Innenwandungen 80a und/oder den Halsbereich 62a eine Beschädigung aufzuweisen. Die beiden Wandungen der Außenwandung 40a begrenzen Leerräume 82a. Die Leerräume 82a sind mit Luft gefüllt. Alternativ könnten die Leerräume 82a mit einem von Luft verschiedenen Gas, beispielsweise Helium, gefüllt sein.

Der Halsbereich 62a und die Seitenfläche 76a sind durch eine Nut 84a verbunden. Die Nut 84a stellt einen spaltenlosen Übergang der Außenwandung 40a von der Aufnahmeeinheit 26a zu der Verschlusseinheit 28a bereit. Der weitere Probenbehälter 16a weist vier Sollbrucheinheiten 30a auf. Die Sollbrucheinheiten 30a spannen zwischen sich jeweils einen 90°-Winkel auf.

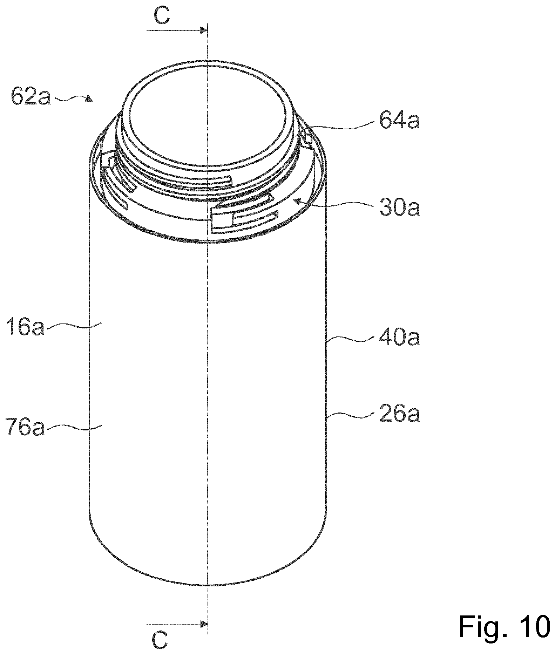

Fig. 10 bis 12 zeigen den weiteren Probenbehälter 16a von außen. Der weitere Probenbehälter weist ein Formschlusselement 42a auf. Das Formschlusselement 42a ist als eine sechseckige Vertiefung ausgebildet. Der weitere Probenbehälter 16a weist ein weiteres Formschlusselement 44a auf. Das weitere Formschlusselement 44a ist als eine sechseckige Erhebung ausgebildet.

### Bezugszeichen

- 10: Öffnungsmaschine
- 11: Schalter
- 12: Öffnungsmaschinenvorrichtung
- 14: Öffnungseinheit
- 16: Probenbehälter
- 18: Verifikationseinheit
- 20: Optische Sensoreinheit
- 22: Belastungssensoreinheit
- 24: Ausleseeinheit
- 26: Aufnahmeeinheit
- 28: Verschlusseinheit
- 30: Sollbrucheinheit
- 32: Drehachse
- 34: Sollbruchkante
- 36: Sehhilfe
- 38: Markierung
- 40: Außenwandung
- 42: Formschlusselement
- 44: Formschlusselement
- 46: System
- 48: Halteelement
- 50: Ausfahrelement
- 52: Öffnungselement
- 54: Ausfahrelement
- 55: Kappe
- 56: Plattform
- 58: Haltestange
- 60: Kamera
- 61: RFID-Tag
- 62: Halsbereich
- 63: Boden
- 64: Gewinde
- 66: Gewinde
- 70: Sollbruchelement
- 72: Innenwand
- 74: Rastelement
- 76: Seitenfläche
- 78: Oberseite
- 80: Innenwandung
- 82: Leerraum
- 84: Nut
- 100: Verfahrensschritt
- 110: Verfahrensschritt
- 120: Verfahrensschritt
- 130: Verfahrensschritt
- 140: Verfahrensschritt
- 150: Verfahrensschritt
- 160: Verfahrensschritt

## Patentansprüche

1. Öffnungsmaschinenvorrichtung (12) mit zumindest einer Öffnungseinheit (14) zur automatischen Öffnung zumindest eines Probenbehälters (16; 16a) und mit einer Recheneinheit, wobei zumindest eine Verifikationseinheit (18) dazu vorgesehen ist, zumindest eine Verifikationseigenschaft des Probenbehälters (16; 16a) automatisch zu überprüfen, wobei die Verifikationseigenschaft eine Eigenschaft des Probenbehälters (16; 16a) ist, welche zumindest eine Information bezüglich einer Unversehrtheit des Probenbehälters (16; 16a) und/oder einer Unversehrtheit einer Probe aufweist und wobei die Verifikationseinheit (18) zumindest eine Belastungssensoreinheit (22) aufweist, welche dazu vorgesehen ist, zumindest eine mechanische Verifikationseigenschaft des Probenbehälters (16; 16a) zu detektieren, **dadurch gekennzeichnet, dass** die Belastungssensoreinheit (22) einen Drehmomentsensor aufweist und die mechanische Verifikationseigenschaft des Probenbehälters (16; 16a) das zu einer Rotation der Verschlusseinheit (28) relativ zur Aufnahmeeinheit (26) benötigte Drehmoment definiert, wobei die Belastungssensoreinheit (22) eine Übertragungseinheit aufweist, welche zu einer Übertragung des steigenden Drehmoments an die Recheneinheit dient, wobei die Recheneinheit das gemessene Drehmoment stetig mit einem vordefinierten Drehmoment vergleicht und, sobald beide Drehmomente identisch sind, ein Signal zur Freigabe der Öffnung des Probenbehälters (16; 16a) an die Öffnungseinheit (14) überträgt.

2. Öffnungsmaschinenvorrichtung (12) nach Anspruch 1, **dadurch gekennzeichnet, dass** die Verifikationseinheit (18) zumindest eine optische Sensoreinheit (20) aufweist, welche dazu vorgesehen ist, zumindest eine optische Verifikationseigenschaft des Probenbehälters (16; 16a) zu detektieren.

3. Öffnungsmaschinenvorrichtung (12) nach Anspruch 1 oder 2, **dadurch gekennzeichnet, dass** die Belastungssensoreinheit (22) zumindest teilweise einstückig mit der Öffnungseinheit (14) ausgeführt ist.

4. Öffnungsmaschinenvorrichtung (12) nach einem der vorhergehenden Ansprüche, **dadurch gekennzeichnet, dass** die Verifikationseinheit (18) zumindest eine Ausleseeinheit (24) aufweist, welche dazu vorgesehen ist, zumindest eine elektronische Verifikationseigenschaft des Probenbehälters (16; 16a) zu detektieren.

5. Öffnungsmaschine (10) mit einer Öffnungsmaschinenvorrichtung (12) nach einem der vorhergehenden Ansprüche.

6. System (46) mit zumindest einer Öffnungsmaschinenvorrichtung (12) nach einem der Ansprüche 1 bis 4 und mit zumindest einem Probenbehälter (16), welcher zumindest eine Aufnahmeeinheit (26; 26a), wenigstens eine mit der Aufnahmeeinheit (26; 26a) in zumindest einem verschlossenen Zustand kooperierende Verschlusseinheit (28; 28a) und zumindest eine Außenwandung (40; 40a) aufweist.

7. Verfahren mit einer Öffnungsmaschinenvorrichtung (12), bei welchem zumindest ein Probenbehälter (16; 16a) automatisch geöffnet wird, wobei mittels zumindest einer Verifikationseinheit (18) zumindest eine Verifikationseigenschaft des Probenbehälters (16) automatisch überprüft wird, wobei die Verifikationseigenschaft eine Eigenschaft des Probenbehälters (16; 16a) ist, welche zumindest eine Information bezüglich einer Unversehrtheit des Probenbehälters (16; 16a) und/oder einer Unversehrtheit einer Probe aufweist und wobei die Verifikationseinheit (18) zumindest eine Belastungssensoreinheit (22) aufweist, mittels der zumindest eine mechanische Verifikationseigenschaft des Probenbehälters (16; 16a) detektiert wird, **dadurch gekennzeichnet, dass** die Belastungssensoreinheit (22) einen Drehmomentsensor aufweist und die mechanische Verifikationseigenschaft des Probenbehälters (16; 16a) das zu einer Rotation der Verschlusseinheit (28) relativ zur Aufnahmeeinheit (26) benötigte Drehmoment definiert, wobei die Belastungssensoreinheit (22) eine Übertragungseinheit aufweist, mittels der eine Übertragung des steigenden Drehmoments an eine Recheneinheit erfolgt, wobei die Recheneinheit das gemessene Drehmoment stetig mit einem vordefinierten Drehmoment vergleicht und, sobald beide Drehmomente identisch sind, ein Signal zur Freigabe der Öffnung des Probenbehälters (16; 16a) an die Öffnungseinheit (14) übertragen wird.

## Claims

1. Opening machine device (12)
with at least one opening unit (14) for an automatic opening of at least one sample container (16; 16a) and with a computing unit,
wherein at least one verification unit (18) is configured to automatically check at least one verification characteristic of the sample container (16; 16a),
the verification characteristic being a characteristic of the sample container (16; 16a) that contains at least one piece of information concerning an integrity of the sample container (16; 16a) and/or an integrity of a sample, and
the verification unit (18) comprising at least one load sensor unit (22), which is configured to detect at least one mechanical verification characteristic of the sample container (16; 16a),
**characterised in that** the load sensor unit (22) comprises a torque sensor and the mechanical verification characteristic of the sample container (16; 16a) defines the torque that is required for a rotation of the closure unit (28) relative to the receiving unit (26),
wherein the load sensor unit (22) comprises a transmission unit which serves for a transmission of the increasing torque to the computing unit,
wherein the computing unit continuously compares the measured torque to a predefined torque and, as soon as the two torques are identical, transmits a signal for a release of the opening of the sample container (16; 16a) to the opening unit (14).

2. Opening machine device (12) according to claim 1,
**characterised in that** the verification unit (18) comprises at least one optical sensor unit (20), which is configured to detect at least one optical verification characteristic of the sample container (16; 16a).

3. Opening machine device (12) according to claim 1 or 2,
**characterised in that** the load sensor unit (22) is at least partially realized integrally with the opening unit (14).

4. Opening machine device (12) according to one of the preceding claims,
**characterised in that** the verification unit (18) comprises at least one read-out unit (24), which is configured to detect at least one electronic verification characteristic of the sample container (16; 16a).

5. Opening machine (10) with an opening machine device (12) according to one of the preceding claims.

6. System (46) with at least one opening machine device (12) according to one of claims 1 to 4, and with at least one sample container (16) that comprises
at least one receiving unit (26; 26a),
at least one closure unit (28; 28a) that cooperates with the receiving unit (26; 26a) in
at least one closed state, and
at least one outer wall (40; 40a).

7. Method with an opening machine device (12),
in which at least one sample container (16; 16a) is opened automatically, wherein at least one verification characteristic of the sample container (16) is checked automatically by means of at least one verification unit (18),
wherein the verification characteristic is a characteristic of the sample container (16; 16a) which contains at least one piece of information concerning an integrity of the sample container (16; 16a) and/or an integrity of a sample, and
wherein the verification unit (18) comprises at least one load sensor unit (22), by means of which at least one mechanical verification characteristic of the sample container (16; 16a) is detected,
**characterised in that** the load sensor unit (22) comprises a torque sensor and the mechanical verification characteristic of the sample container (16; 16a) defines the torque that is required for a rotation of the closure unit (28) relative to the receiving unit (26),
wherein the load sensor unit (22) comprises a transmission unit via which a transmission of the increasing torque to a computing unit is realized,
wherein the computing unit continuously compares the measured torque with a predefined torque and, as soon as the two torques are identical, a signal for a release of the opening of the sample container (16; 16a) is transmitted to the opening unit (14).

## Revendications

1. Dispositif d'appareil ouvreur (12),
avec au moins une unité ouvreuse (14) pour une ouverture automatique d'au moins un récipient à échantillons (16 ; 16a) et avec une unité calculatrice,
où au moins une unité de vérification (18) est prévué pour vérifier automatiquement au moins une caractéristique à vérification du récipient à échantillons (16 ; 16a), la caractéristique à vérification étant une caractéristique du récipient à échantillons (16 ; 16a) qui comprend au moins une information concernant une intégrité du récipient à échantillons (16 ; 16a) et/ou une intégrité d'un échantillon, et
où l'unité de vérification (18) comprend au moins une unité captrice de charge (22), qui est prévue pour détecter au moins une caractéristique mécanique à vérification du récipient à échantillons (16 ; 16a),
**caractérisé en ce que** l'unité captrice de charge (22) comporte un capteur de couple et la caractéristique mécanique à vérification du récipient à échantillons (16 ; 16a) définit le couple requis pour une rotation de l'unité de fermeture (28) par rapport à l'unité réceptrice (26),
l'unité captrice de charge (22) comprenant une unité transmettrice qui sert pour une transmission du couple croissant à l'unité calculatrice,
où l'unité calculatrice compare le couple mesuré continuellement à un couple prédéfini et, aussitôt que les deux couples soient identiques, transmet à l'unité ouvreuse (14) un signal pour un déblocage du récipient à échantillons (16 ; 16a).

2. Dispositif d'appareil ouvreur (12) selon la revendication 1,
**caractérisé en ce que** l'unité de vérification (18) comporte au moins une unité captrice optique (20) qui est prévue pour détecter au moins une caractéristique optique à verification du récipient à échantillons (16 ; 16a).

3. Dispositif d'appareil ouvreur (12) selon la revendication 1 ou 2,
**caractérisé en ce que** l'unité captrice de charge (22) est au moins partiellement réalisée intégralement avec l'unité ouvreuse (14).

4. Dispositif d'appareil ouvreur (12) selon l'une des revendications précédentes,
**caractérisé en ce que** l'unité de vérification (18) comporte au moins une unité de lecture (24) qui est prévue pour détecter au moins une caractéristique électronique à vérification du récipient à échantillons (16 ; 16a).

5. Appareil ouvreur (10) avec un dispositif d'appareil ouvreur (12) selon l'une des revendications précédentes.

6. Système (46) avec au moins un dispositif d'appareil ouvreur (12) selon l'une des revendications 1 à 4, et avec au moins un récipient à échantillons (16) comprenant
au moins une unité réceptrice (26 ; 26a),
au moins une unité de fermeture (28 ; 28a) coopérant avec l'unité réceptrice (26 ; 26a) dans au moins un état fermé, et
au moins une paroi extérieure (40 ; 40a).

7. Procédé avec un dispositif d'appareil ouvreur (12),
dans lequel au moins un récipient à échantillons (16 ; 16a) est ouvert automatiquement,
où au moins une caractéristique à vérification du récipient à échantillons (16) est vérifiée automatiquement par au moins une unité de vérification (18),
la caractéristique à vérification étant une caractéristique du récipient à échantillons (16 ; 16a) qui comprend au moins une information concernant une intégrité due récipient à échantillons (16 ; 16a) et/ou une intégrité d'un échantillon
et l'unité de vérification (18) comportant au moins une unité captrice de charge (22), par le biais de laquelle au moins une caractéristique mécanique à vérification du récipient à échantillons (16 ; 16a) est détectée,
**caractérisé en ce que** l'unité captrice de charge (22) comporte un capteur de couple et la caractéristique mécanique à vérification du récipient à échantillons (16 ; 16a) définit le couple requis pour une rotation de l'unité de fermeture (28) par rapport à l'unité réceptrice (26),
l'unité captrice de charge (22) comportant une unité de transmission par laquelle une transmission du couple croissant à une unité calculatrice est effectuée, où l'unité calculatrice compare le couple mesuré continuellement à un couple prédéfini et, aussitôt que les deux couples soient identiques, un signal pour un déblocage du récipient à échantillons (16 ; 16a) est transmis à l'unité ouvreuse (14).
